(19) Europäisches Patentamt
European Patent Office
Office européen des brevets

(11) Publication number: **0 093 488 A2**

(12) **EUROPEAN PATENT APPLICATION**

(21) Application number: 83301158.8

(22) Date of filing: 04.03.83

(51) Int. Cl.³: C 07 D 471/08
A 61 K 31/44
//(C07D471/08, 221/00, 221/00)

(30) Priority: 18.03.82 GB 8207867

(43) Date of publication of application: 09.11.83 Bulletin 83/45

(84) Designated Contracting States: BE CH DE FR GB IT LI NL SE

(71) Applicant: BEECHAM GROUP PLC
Beecham House Great West Road
Brentford Middlesex(GB)

(72) Inventor: Hadley, Michael Stewart
9 Coney Gree
Sawbridgeworth Hertfordshire(GB)

(74) Representative: Stott, Michael John et al,
European Patent Attorney Beecham Pharmaceuticals
Great Burgh Yew Tree Bottom Road
Epsom Surrey KT18 5XQ(GB)

(54) **Nortropyl benzopyrrolinone compounds, process for their preparation and pharmaceutical compositions containing them.**

(57) Compounds of formula (I) and pharmaceutically acceptable salt and/or solvates and/or N-oxides thereof:

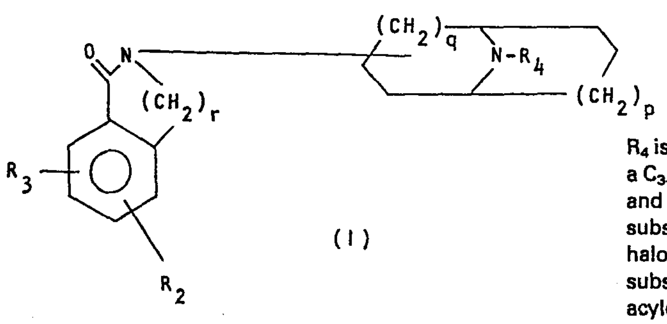

(I)

wherein:

$R_2$ and $R_3$ are the same or different and are hydrogen, halogen, $CF_3$, $C_{1-6}$ alkyl, $C_{1-7}$ acyl, $C_{1-7}$ acylamino or amino, aminocarbonyl or aminosulphonyl optionally substituted by one or two $C_{1-6}$ alkyl, $C_{3-8}$ cycloalkyl, $C_{3-8}$ cycloalkyl, $C_{1-4}$ alkyl, phenyl or phenyl $C_{1-4}$ alkyl groups either of which phenyl moieties may be substituted by one or more halogen, trifluoromethyl, $C_{1-6}$ alkoxy or nitro groups or N-substituted by $C_{4-5}$ polymethylene, $C_{1-6}$ alkylsulphonyl, $C_{1-6}$ alkylsulphinyl, $C_{1-6}$ alkoxy $C_{1-6}$ alkylthio; hydroxy or nitro; or $R_2$ and $R_3$ taken together are methylenedioxy or ethylenedioxy;

$R_4$ is $C_{1-7}$ alkyl or a group $-(CH_2)_sR_6$ where s is 0 to 2 and $R_6$ is a $C_{3-8}$ cycloalkyl group, or a group $-(CH_2)_tR_7$ where t is 1 or 2 and $R_7$ is a phenyl group optionally substituted by one or two substituents selected from $C_{1-4}$ alkoxy, trifluoromethyl and halogen, carboxy, esterified carboxy, or $C_{1-4}$ alkyl optionally substituted by hydroxy, $C_{1-4}$ alkoxy in vivo hydrolysable acyloxy, carboxy, or esterified carboxy; or a thienyl group; p and q are independently 0 to 2; and r is 1 or 2. having dopamine antagonist activity, a process to their preparation and their use.

EP 0 093 488 A2

Croydon Printing Company Ltd.

0093488

PJ/B1225

TITLE MODIFIED
see front page

NOVEL COMPOUNDS

This invention relates to novel compounds having useful pharmacological properties, in particular dopamine antagonist activity, to pharmaceutical compositions containing them, and to a process for their preparation.

European Patent Application No. 79302978.6 discloses that compounds of the formula (A), and pharmaceutically acceptable salts thereof:

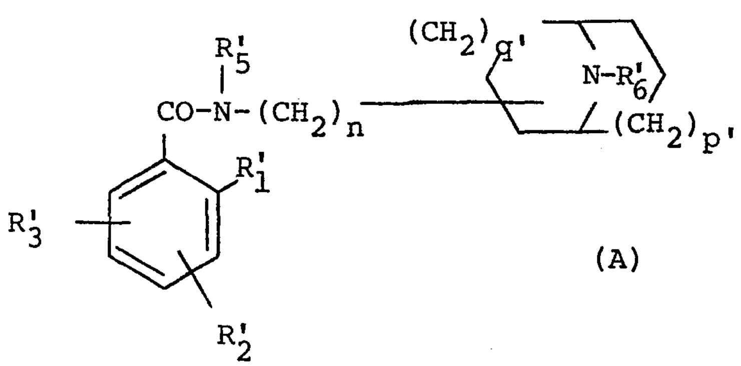

(A)

wherein:

$R_1'$ is a $C_{1-6}$ alkoxy group;

$R_2'$ and $R_3'$ are the same or different and are hydrogen, halogen, $CF_3$, $C_{2-7}$ acyl, $C_{2-7}$ acylamino, or amino, aminocarbonyl or aminosulphone optionally substituted by one or two $C_{1-6}$ alkyl groups, $C_{1-6}$ alkylsulphone or nitro;

$R_5'$ is hydrogen or $C_{1-6}$ alkyl;

$R_6'$ is $C_{1-7}$ alkyl or a group $-(CH_2)_sR_7'$ where s is 0 to 2 and $R_7'$ is a $C_{3-8}$ cycloalkyl group, or a group $-(CH_2)_{t'}R_8'$ where t' is 1 or 2 and $R_8'$ is $C_{2-5}$ alkenyl or a phenyl group optionally substituted by one or two substituents selected from $C_{1-6}$ alkyl, $C_{1-4}$ alkoxy, trifluoromethyl and halogen; and

n, p' and q' are independently 0 to 2; have useful pharmacological activity. More specifically the compounds of formula (A) are stated to be useful in the treatment of disorders related to impaired gastro-intestinal motility and/or in the treatment of disorders of the central nervous system. All the compounds are stated to have anti-emetic activity.

European Patent Application No. 80850104.3 discloses compounds of the formula (B):

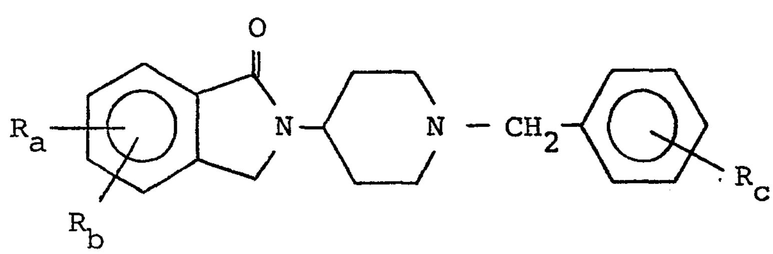

(B)

wherein:

$R_a$, $R_b$ and $R_c$ are the same or different and are each selected from hydrogen, halogen, alkyl having 1, 2 or 3 carbon atoms, and trifluoromethyl; which compounds have antipsychotic activity.

A structurally distinct group of compounds have now been discovered, which compounds have dopamine antagonist activity.

Accordingly, the present invention provides compounds of the formula (I) or a pharmaceutically acceptable salt and/or solvate and/or N-oxide thereof:

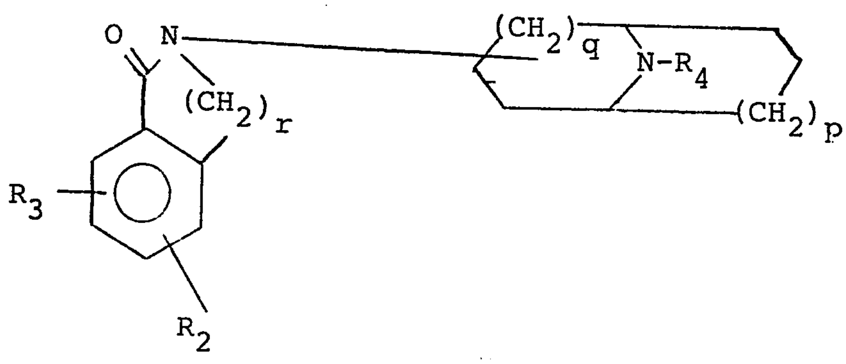

(I)

wherein:

$R_2$ and $R_3$ are the same or different and are hydrogen, halogen, $CF_3$, $C_{1-6}$ alkyl, $C_{1-7}$ acyl, $C_{1-7}$ acylamino or amino, aminocarbonyl or aminosulphonyl optionally substituted by one or two $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups either of which phenyl moieties may be substituted by one or more halogen, trifluoromethyl, $C_{1-6}$ alkoxy or nitro groups or N-substituted by $C_{4-5}$ polymethylene,

$C_{1-6}$ alkylsulphonyl, $C_{1-6}$ alkylsulphinyl, $C_{1-6}$ alkoxy $C_{1-6}$ alkylthio; hydroxy or nitro; or $R_2$ and $R_3$ taken together are methylenedioxy or ethylenedioxy;

$R_4$ is $C_{1-7}$ alkyl or a group $-(CH_2)_sR_6$ where s is 0 to 2 and $R_6$ is a $C_{3-8}$ cycloalkyl group, or a group $-(CH_2)_tR_7$ where t is 1 or 2 and $R_7$ is a phenyl group optionally substituted by one or two substituents selected from $C_{1-4}$ alkoxy, trifluoromethyl and halogen, carboxy, esterified carboxy, or $C_{1-4}$ alkyl optionally substituted by hydroxy, $C_{1-4}$ alkoxy in vivo hydrolysable acyloxy, carboxy, or esterified carboxy; or a thienyl group;

p and q are independently 0 to 2; and

r is 1 or 2.

Suitable examples of the groups $R_2$ and $R_3$ include the following groups: hydrogen, chlorine, bromine, amino, $C_{1-4}$ alkanoylamino such as formylamino, acetylamino, propionylamino, n- and iso-butyrlamino, aminosulphonyl, and amino and aminosulphonyl substituted by one or two methyl, ethyl, n- or iso-propyl, or cyclopentyl groups or by tetramethylene, nitro, methoxy, ethoxy, n- and iso-propoxy, methylthio, ethylthio, n- and iso-propylthio, hydroxy, methyl,ethyl- and isopropyl-sulphonyl.

Particularly suitable $R_2$ and $R_3$ groups include hydrogen, halogen, methoxy, optionally substituted amino and aminosulphonyl as defined and methylsulphonyl.

When $R_2$ and $R_3$ are other than $C_{1-6}$ alkoxy or $C_{1-6}$ alkylthio, and r=1, it is generally preferred that $R_2$ is in the 6-position (see formula (X))for greater activity in the resulting compound of the formula (I). For the same reason, it is generally preferred than $R_3$ is in the 5-position. When one of $R_2$ and $R_3$ is $C_{1-6}$ alkoxy, it is preferably methoxy and the other is hydrogen. Particularly preferred $R_3$ groups include 5- halo, such as 5-bromo and 5-chloro, and 5-amino. $R_2$ groups of interest include optionally substituted 6-aminosulphonyl as defined and 6-$C_{1-6}$ alkylsulphonyl or -sulphinyl, such as 6-aminosulphonyl and 6-methylsulphonyl.

When $R_3$ and $R_2$ taken together are methylenedioxy or ethylenedioxy $R_3$ and $R_2$ are preferably ethylenedioxy.

Often the pyrrolinone and heterocycle nitrogen atoms are separated by 2 or 3 carbon atoms, preferably 3.

Suitable examples of $R_4$ when $C_{1-7}$ alkyl include methyl, ethyl, n- and iso- and n-, sec- and tert-butyl. Within $C_{1-7}$ alkyl, $C_{4-7}$ alkyl are of interest, especially those of the formula $(CH_2)_uR_{16}$ wherein u is 1 or 2 and $R_{16}$ is a secondary or tertiary $C_{3-6}$ alkyl group. Examples of $C_{4-7}$ alkyl include n-, sec- and tert-butyl, n-pentyl, n-heptyl and especially iso-butyl, 3-methylbutyl, 2,2-dimethylpropyl and 3,3-dimethylbutyl.

When $R_4$ is a group $-(CH_2)_sR_6$ as defined, suitable examples of $R_6$ include $C_{5-8}$ cycloalkyl, preferably cyclohexyl. s is preferably 1.

When $R_4$ is a group $-(CH_2)_tR_7$ as defined, t is preferably 1.

When $R_7$ is optionally substituted phenyl as defined above, suitable examples of such optional phenyl substituents include $CF_3$, fluoro, chloro or bromo.

Other $R_7$ substituents may be represented as $L-R_{10}$ wherein L is $C_{1-4}$ alkylene and $R_{10}$ is hydrogen, hydroxyl, in vivo hydrolysable acyloxy, carboxy or esterified carboxy, or L is a bond and $R_{10}$ is carboxy or esterified carboxy, or $C_{1-4}$ alkoxy.

Examples of L include a bond, methylene, ethane-1, 2-diyl, propane-1, 3-diyl, propane-1, 2-diyl, butane-1, 4-diyl, butane-1, 3-diyl and 2-methylpropane-1, 3-diyl, preferably methylene.

Examples of $R_{10}$ include hydrogen; hydroxyl; $C_{1-4}$ alkoxy such as methoxy, ethoxy, n- and iso-propoxy; in vivo hydrolysable acyloxy groups, such as $C_{1-6}$ alkanoyloxy, for example acetoxy, propionoxy, and n- and iso-butyroxy-2,2-dimethylpropanoyloxy, benzoyloxy optionally substituted in the phenyl ring by one or two substituents selected from $C_{1-4}$ alkyl, $C_{1-4}$ alkoxy, trifluoromethyl, halogen or nitro, and sulphonyloxy groups for example $C_{1-6}$ alkanesulphonyloxy, eg methanesulphonyloxy, or benzenesulphonyloxy optionally substituted as for benzoyloxy; carboxy and carboxy esterified by groups described hereinafter for esterifying carboxy substituents.

When $R_{10}$ is esterified carboxy, suitable esterifying groups include $C_{1-4}$ alkyl, such as methyl, ethyl, n- and iso-propyl; phenyl optionally substituted by one or two substituents selected from $C_{1-4}$ alkyl, $C_{1-4}$ alkoxy, trifluoromethyl, halogen or nitro; and benzyl optionally substituted as for phenyl.

One value of $R_7$ when optionally substituted phenyl is unsubstituted. Preferred optionally substituted phenyl include 4-methylphenyl, 4-fluorophenyl and 4-chlorophenyl.

When $R_7$ is thienyl it may be 2- or 3-thienyl, generally 2-thienyl.

$R_4$ is favourably benzyl, optionally substituted as hereinbefore defined, or 2-thienylmethyl, also called 2-thenyl. Optionally substituted benzyl is preferred, in particular 4-methyl-, 4-chloro and 4-fluorobenzyl.

q is suitably 0 or 1, preferably 1.

p is suitably 0 or 1, preferably 0, particularly preferably 1.

r is preferably 1.

The pharmaceutically acceptable salts of the compound of the formula (I) include acid addition salts with conventional acids such as hydrochloric, hydrobromic, phosphoric, sulphuric, citric, tartaric, lactic and acetic acid.

The pharmaceutically acceptable salts of the compounds of the formula (I) also include quaternary ammonium salts. Examples of such salts include such compounds quaternised by compounds such as $R_8$ - Y wherein $R_8$ is $C_{1-6}$ alkyl, phenyl-$C_{1-6}$ alkyl or $C_{5-7}$ cycloalkyl, and Y is a radical corresponding to an anion of an acid. Suitable examples of $R_8$ include methyl, ethyl and n- and iso-propyl; and benzyl and phenethyl. Suitable examples of Y include the halides such as chloride, bromide and iodide.

Examples of pharmaceutically acceptable salts also include internal salts such as N-oxides.

The compounds of the formula (I) may also form solvates such as hydrates and the invention extends to such solvates.

There is a group of compounds within formula (I) wherein $R_2$ and $R_3$ are the same or different and are hydrogen, halogen, $CF_3$, $C_{1-6}$alkyl, $C_{1-7}$acyl, $C_{1-7}$ acylamino or amino, aminocarbonyl or aminosulphonyl optionally substituted by one or two $C_{1-6}$ alkyl or $C_{3-8}$ cycloalkyl groups, or by $C_{4-5}$ polymethylene, $C_{1-6}$ alkylsulphonyl, $C_{1-6}$ alkylsulphinyl, $C_{1-6}$ alkoxy, $C_{1-6}$ alkylthio; hydroxy or nitro; or $R_3$ and $R_2$ taken together are methylenedioxy or ethylenedioxy; and the remaining variables are as defined in formula (I).

From the aforesaid it will be seen that suitably the moiety of formula (II):

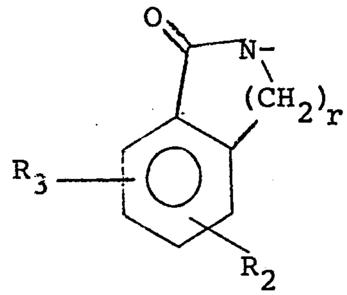

(II)

in a compound of the formula (I) may have the structure (III):

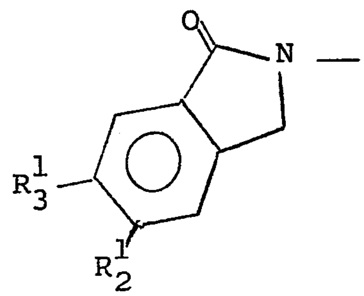

(III)

wherein $R^1_2$ is hydrogen, halogen or amino, and $R^1_3$ is hydrogen or halogen.

A preferred group of compounds within those of formula (I), are those of formula (IV):

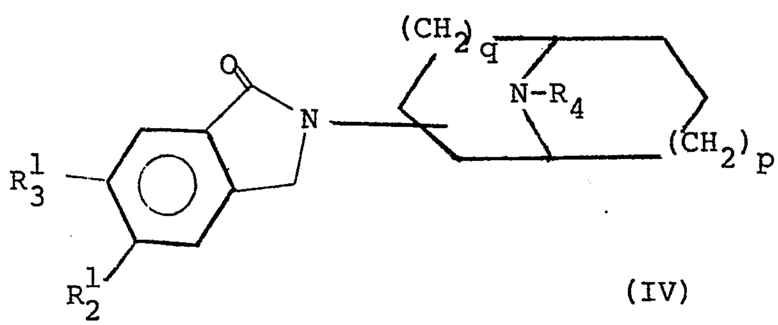

(IV)

wherein $R_4$, $R^1_2$, $R^1_3$, p and q are as defined in formulæ (I) and (III).

More suitably p is 0 or 1. Preferably q is 1 and the moiety of formula (II) is then attached at the 3 position (standard numbering).

Suitable and preferred examples of $R_4$ in formula (IV) include those listed under formula (I) for $R_4$. Particularly preferred examples of $R_4$ include benzyl optionally substituted in the phenyl ring as defined under formula (I). Unsubstituted benzyl, 4-methyl-, 4-fluoro- and 4-chlorobenzyl are included in preferred $R_4$.

Preferably $R_2^1$ is hydrogen, bromo, chloro or amino and $R_3^1$ is hydrogen; or $R_2^1$ is amino and $R_3^1$ is chloro.

A sub-group of compounds within those of formula (IV) are those of the formula (V):

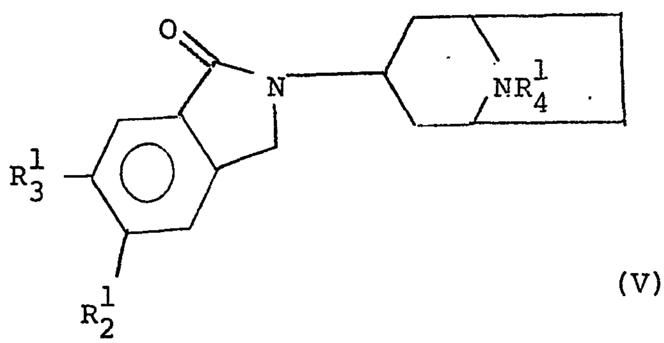

(V)

wherein $R_4^1$ is $C_{4-7}$ alkyl or $-(CH_2)_sR_9$ where s is 0 to 2 and $R_9$ is $C_{5-8}$ cycloalkyl; and $R_2^1$ and $R_3^1$ as hereinbefore defined.

Suitable examples of $R_4^1$ are as so described for $R_4$ $C_{4-7}$ alkyl under formula (I).

It is preferred that the pyrrolinone moiety is in the β-orientation to the nortropane ring, that is as follows:

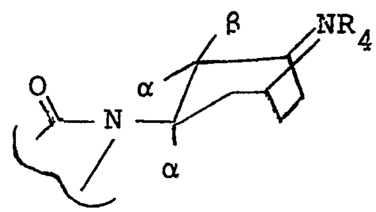

(The 2α and 2β orientations are also depicted.)

A preferred sub-group of compounds within those of formula (IV) are those of the formula (VI):

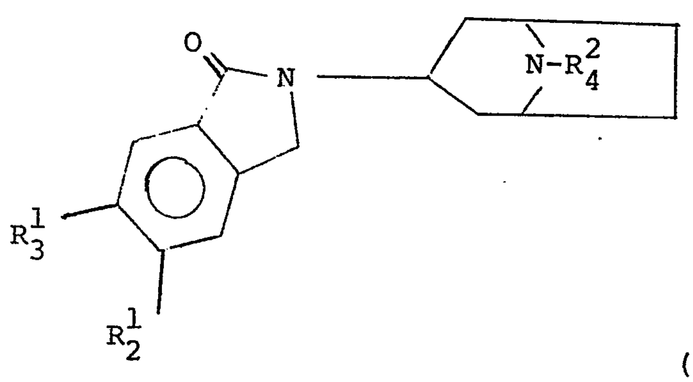

(VI)

wherein $R_4^2$ is a group $-(CH_2)_tR^1_7$ wherein t is 1 or 2 and $R_7^1$ is optionally substituted phenyl as defined in formula (I); or 2-thienylmethyl; and $R_2^1$ and $R_3^1$ is as hereinbefore defined.

Suitable and preferred $R_4^2$ are as so described for the corresponding $R_4$ groups under formula (I).

Benzyl, 4-methylbenzyl, 4-fluorobenzyl and 4-chlorobenzyl are included in preferred $R_4^2$ values.

It is preferred that the pyrrolinone moiety is in the β-orientation to the nortropane ring.

A further sub-group of compounds within those of the formula (IV) of interest are those of the formula (VII):

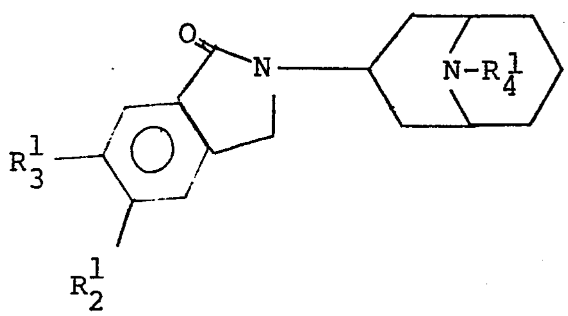

(VII)

wherein $R_4^1$,$R_2^1$ & $R_3^1$ are as defined in formula (V).

Suitable and preferred $R_4^1$ are as described under formula (V).

It is preferred that the pyrrolinone moiety is in the β-orientation to the granatane ring, the β-orientation being the same as in the nortropane hereinbefore depicted.

A particularly preferred sub-group of compounds within those of the formula (IV) are those of the formula (VIII):

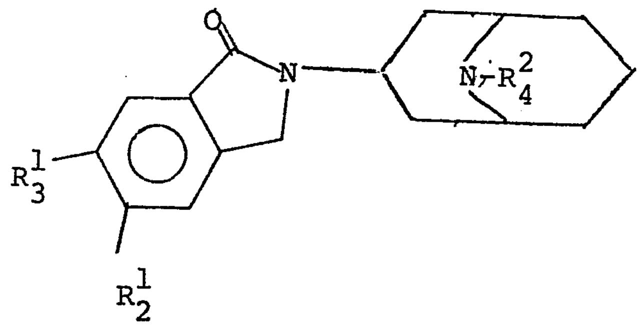

(VIII)

wherein $R_4^2$, $R_2^1$ & $R_3^1$ are as defined in formula (VI).

Suitable and preferred examples of $R_4^2$ are as described under formula (VI).

It is preferred that the pyrrolinone moiety is in the β-orientation to the granatane ring.

A second group of compounds within those of the formula (I) which is of interest is of the formula (IX):

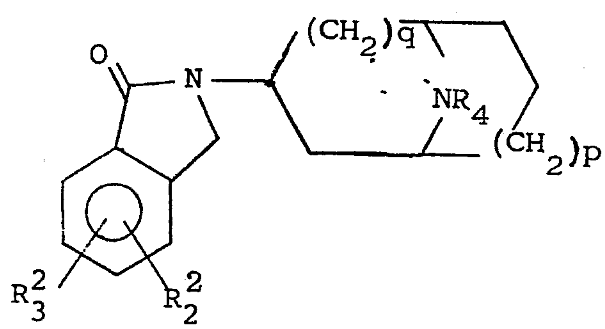

(IX)

wherein:

$R_2^2$ and $R_3^2$ are the same or different and are hydrogen, aminosulphonyl optionally substituted by one or two $C_{1-6}$ alkyl groups, $C_{1-6}$ alkylsulphonyl, $C_{1-6}$ alkylsulphinyl, $C_{1-6}$ alkoxy or hydroxy with the proviso that $R_2^2$ and $R_3^2$ are not both hydrogen, or $R_3^2$ and $R_2^2$ taken together are methylenedioxy; and $R_4$ is as defined in formula (I).

It is preferred that the pyrrolinone moiety is in the β-orientation to the bicycle.

One of $R_2^2$ and $R_3^2$ is preferably hydrogen, and the other is then preferably methoxy.

Suitable and preferred $R_4$ are as so described under formula (VI).

A sub-group of compounds within those of formula (IX) are those of the formula (X):

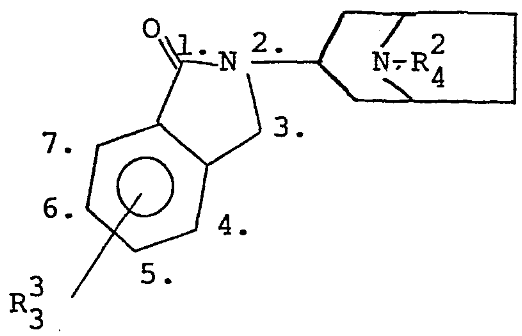

(X)

wherein $R_3^3$ is aminosulphonyl optionally substituted by one or two $C_{1-6}$ alkyl groups or $C_{1-6}$ alkylsulphonyl or $C_{1-6}$ alkylsulphinyl and $R_4^2$ as hereinbefore defined.

$R_3^3$ is preferably aminosulphonyl or methylsulphonyl, in particular 6-aminosulphonyl or 6-methylsulphonyl as shown

Suitable and preferred $R_4^2$ are as so described under formula (VI).

There are further groups and sub-groups of compounds within formula (I) corresponding to those of formulae (IV) to (X) respectively wherein r is 2 and the remaining variables are as defined therein.

It will of course be realised that the compounds of the formula (I) have chiral or prochiral centres, and thus are capable of existing in a number of stereoisomeric forms. The invention extends to each of these stereoisomeric forms, and to mixtures thereof (including racemates). The different stereoisomeric forms may be separated one from the other by the usual methods, or any given isomer may be obtained by stereospecific or asymmetric synthesis.

The invention also provides a process for the preparation of a compound of the formula (I), which process comprises reacting an acid derivative of the formula (XI):

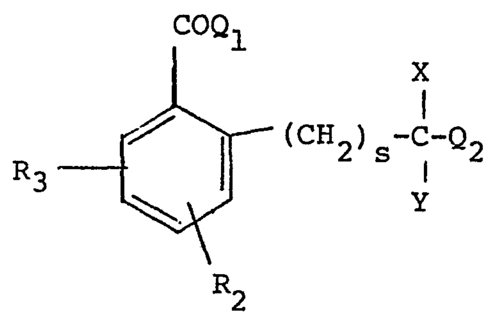

(XI)

wherein X is H and Y is H; or

X and Y together form an oxo group;

$Q_1$ and $Q_2$ are independently leaving groups; or together are O; s is 0 or 1; and the remaining variables are as in formula (I), with a compound of formula (XII);

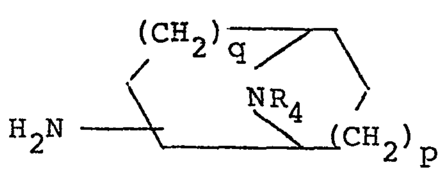

(XII)

wherein the variables are as defined in formula (I); and thereafter as necessary reducing X and Y when an oxo group to X and Y both hydrogen; and if desired or necessary converting a group $R_2$ or $R_3$ in the thus formed compound to another group $R_2$ or $R_3$ respectively; converting $R_4$ when hydrogen to another $R_4$; and optionally forming a pharmaceutically acceptable salt of the resultant compound of the formula (I).

The leaving groups $Q_1$ and $Q_2$ are groups readily displaceable by a nucleophile. Suitable values for $Q_1$ and $Q_2$ include halogen, such as chloro or bromo; carboxylic acyloxy, such as $C_{1-4}$ alkanoyloxy; and hydroxy; they may also together be O. Suitable values for $Q_1$, and for $Q_2$ when X and Y together are an oxo group also include activated hydrocarbyloxy, such as pentachlorophenoxy.

Suitable values for $Q_2$ when X and Y are each hydrogen include labile acyloxy such as mesyloxy, tosyloxy, or triflate.

a) When X and Y together are an oxo group, $Q_1$ and $Q_2$ may each independently be halogen, carboxylic acyloxy, hydroxy or activated hydrocarbyloxy; or together may be O, to form an active acylating derivative of phthalic acid. $Q_1$ and $Q_2$ will often be the same.

The reaction will normally be carried out in an inert solvent, such as benzene, toluene, THF or DMF.

These reactions may be carried out at any non-extreme temperature such as $20^{o}$-$100^{o}C$ and more suitably $30^{o}$-$80^{o}C$. The higher reaction temperatures are employed with less active acid derivatives of the formula (XI) whereas the lower temperatures are employed with the more reactive acid derivatives of the formula (XI).

When $Q_1$ and $Q_2$ are halogen or carboxylic acyloxy, the reaction will preferably be in the presence of an acid acceptor.

The acid acceptor is suitably an organic base such as a tertiary amine e.g. triethylamine, trimethylamine, pyridine or picoline, or an inorganic acid acceptor, such as calcium carbonate, sodium carbonate, potassium carbonate. It should also be noted that it is possible to use certain acid acceptors as the inert solvent, for example organic bases.

$Q_1$ and $Q_2$ may also each be hydroxyl and reaction is then effected by reacting the acid (XIV) and the compound (XII)in the presence of a dehydrating catalyst such as a carbodiimide, for example dicyclohexylcarbodiimide.

The subsequent reduction of the product is preferably carried out, with or without isolation of the product, with tin/hydrochloric acid at elevated temperature.

Compounds of the formula (XI) in this case are known compounds or prepared by analogy with known compounds.

b) When X and Y are each H, $Q_1$ may be any of the functions listed above. Suitable $Q_2$ then include halogen, hydroxy and labile acyloxy or $Q_1$ and $Q_2$ may together be O.

For $Q_1$ and $Q_2$ other than together being O, suitable reaction conditions are as for corresponding functions in a) above.

$Q_2$ is preferably halogen or labile acyloxy. $Q_1$ and $Q_2$ will often both be the same halogen.

The intermediate of formula (XI) wherein s=O in this case can be prepared by known methods, such as for example reacting a compound of the formula (XIII):

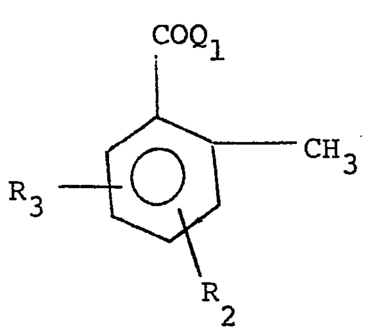

(XIII)

wherein the variables are as defined in formula (XI) with a halogenating agent for electron-deficient methyl such as N-bromosuccinimide, chlorine or bromine, for $Q_2$ halo; base-hydrolysing the product for $Q_2$ hydroxyl and acylating the hydroxyl compound for $Q_2$ labile acyloxy.

Compounds of the formula (XIII) are known or prepared by analogy with known compounds.

When $Q_1$ and $Q_2$ together are O, the reaction is preferably carried out by heating a mixture of the reactants in an inert solvent in a sealed vessel.

Compounds of the formula (XII) are known compounds or can be prepared by analogy to known compounds.

It will be realised that in the compound of the formula (I) the cyclic amide linkage may have an α or β orientation with respect to the ring of the bicyclic moiety to which it is attached. A mixture of α and β isomers of the compound of the formula (I) may be synthesised non-stereospecifically and the desired isomer separated conventionally therefrom, e.g. by chromatography; or alternatively the α or β isomer may if desired be synthesised from the corresponding α or β form of the compound of the formula (XII)

Synthesis from the corresponding α or β isomer of compound of the formula (XII) is in general preferred.

The α or β form of the compound of the formula (XII) may if desired be prepared by known stereospecific processes, such as those leading to the α or β isomers of the compound of the formula (XII) depicted in Scheme 1.

The precursor of the compound of the formula (XII) may be stereospecifically synthesised, such as the azide depicted in Scheme 1, and then converted to the corresponding desired isomer of the compound of the formula (XII)under non-stereospecific conditions with retention of configuration. Alternatively, the precursor may itself have no asymmetric or prochiral centre at the relevant position, but be converted under stereospecific conditions to the desired isomer of the compound of the formula (XII).

Alternatively, a mixture of the α and β isomers of the compound of the formula(XII) may be synthesised non-stereospecifically and the desired isomer separated conventionally therefrom e.g. by chromatography. However, in this case it is generally more convenient to react the mixture to give a mixture of α and β isomers of the compound of the formula (I) and to separate these if desired as hereinbefore described.

The following Scheme 1 illustrates stereospecific and non-stereospecific synthetic routes to intermediates of the formula (XII).

Scheme 1

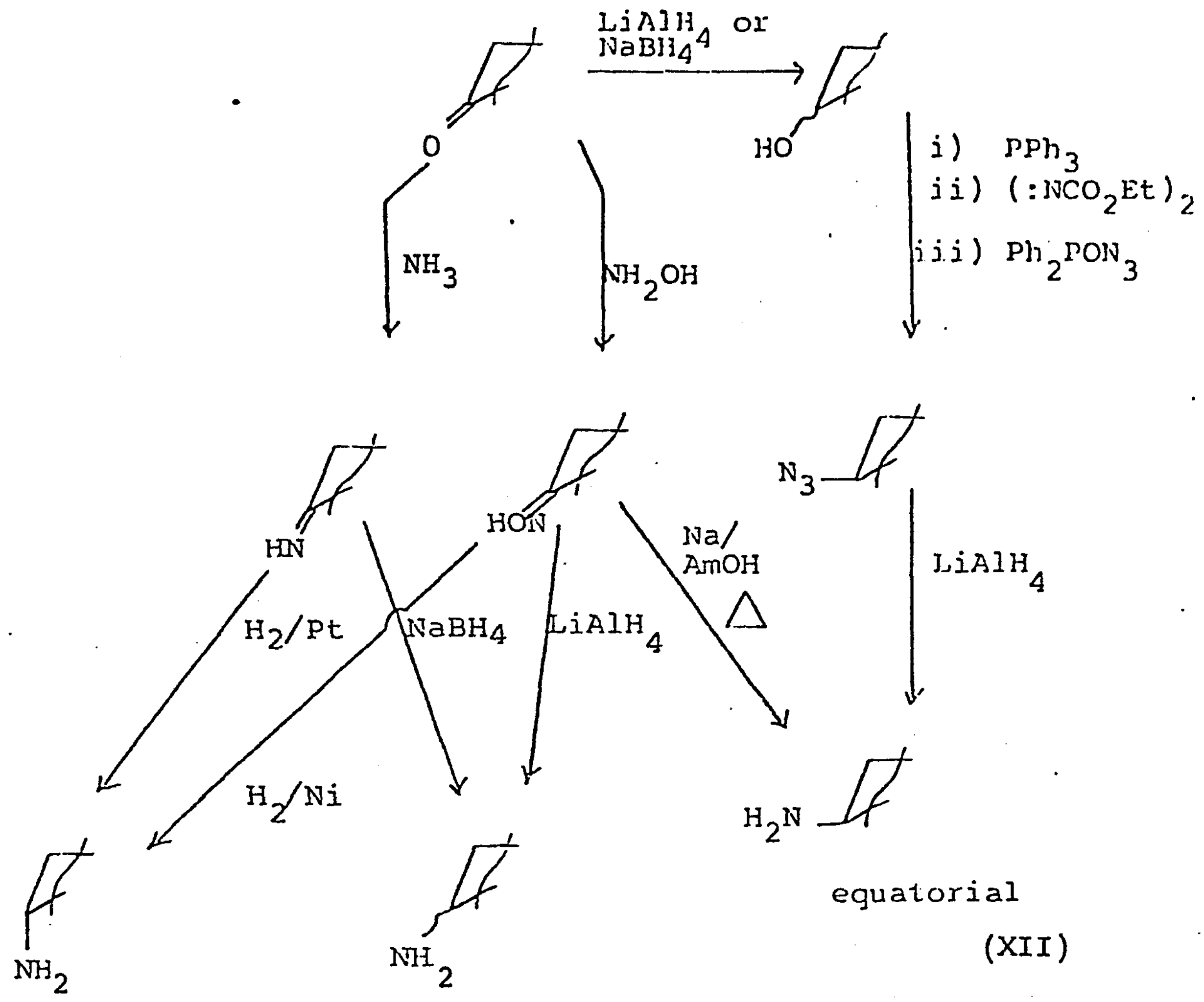


axial (XII)

axial, equatorial (XII)

equatorial (XII)

(Remainder of ring system omitted for clarity)

The skilled man will appreciate that the choice or necessity of conversion of groups $R_2$ and/or $R_3$ to other groups $R_2$ and/or $R_3$ will be dictated by the nature and position of substituents $R_1$, $R_2$ and $R_3$.

It will be apparent that compounds of the formula (I) containing an $R_2$, $R_3$ or $R_4$ group which is convertible to another $R_2$, $R_3$ or $R_4$ group are useful intermediates, and as such form an important aspect of the invention.

By way of example of such conversions, the compounds of the formula (I) wherein $R_2$ and/or $R_3$ is a nitro group meta to the carbonyl function and may be prepared via the nitration of the corresponding intermediate product wherein $R_2$ and/or $R_3$ is a hydrogen atom.

Also the reduction of the intermediates wherein $R_2$ or $R_3$ is a nitro group to $R_2/R_3$ amino may be effected with reagents known to be suitable for reducting nitroarenes to aminoarenes.

Those compounds of the invention wherein $R_2$ or $R_3$ is a $C_{1-7}$ acylamino group may be prepared from the corresponding intermediate wherein $R_2$ or $R_3$ is an amino group by reaction with an acylating derivative, such as previously described as a suitable acylating derivative, e.g. of the acid of the formula (XIV). The reaction may proceed as described for the reaction of the compounds of the formula (XIV) and (XV). For an $R_2/R_3$ formamido group acylation may be effecte d with the free acid.

This invention thus also provides an optional process (I) wherein $R_2$ or $R_3$ is an amino group which process comprises the deacylation of a corresponding intermediate wherein $R_2$ or $R_3$ is a $C_{1-7}$ acylamino group.

Generally the hydrolysis reaction may be effected by treatment with a base such as an alkali metal hydroxide.

Also a compound of the formula (I) wherein $R_2$ or $R_3$ is halogen may be prepared by a conventional halogenation of the corresponding intermediate wherein the said $R_2$ or $R_3$ is hydrogen.

Similarly the compounds wherein $R_2$ or $R_3$ is $C_{1-6}$ alkylthio or $C_{1-6}$ alkylsulphinyl may be oxidised to the corresponding compounds wherein $R_2$ or $R_3$ is $C_{1-6}$ alkylsulphinyl or $C_{1-6}$ alkylsulphonyl respectively.

These oxidations may conveniently be carried out conventionally at below ambient temperatures using an organic peracid in a non-aqueous inert reaction medium preferably a chlorinated hydrocarbon solvent, for example using 3-chloroperbenzoic acid, or using a water soluble inorganic strong oxidant, such as an alkali metal permanganate or hydrogen peroxide in aqueous solution.

It will be appreciated by the skilled man that, depending on the other specific substituents in the compound of the formula (I), such an oxidation on a compound of the formula (I) may also form the N-oxide of the bicyclic moiety therein.

Given the specific substitution desired and it having been decided whether the compound or its N-oxide is required, the skilled man wil readly ascertain whether such $R_2/R_3$ interconversion is desirable. In general it is preferred to effect the oxidation in the intermediate of formula (XIV) before coupling.

It will be appreciated that, when $R_4$ in the compound of the formula (I) is optionally substituted benzyl as hereinbefore defined, $R_4$ may be replaced by another group $R_4$.

Such benzyl groups may be removed for example when $R_2$ or $R_3$ is not halogen by conventional transition metal catalysed hydrogenolysis to give compounds of the formula (XIV):

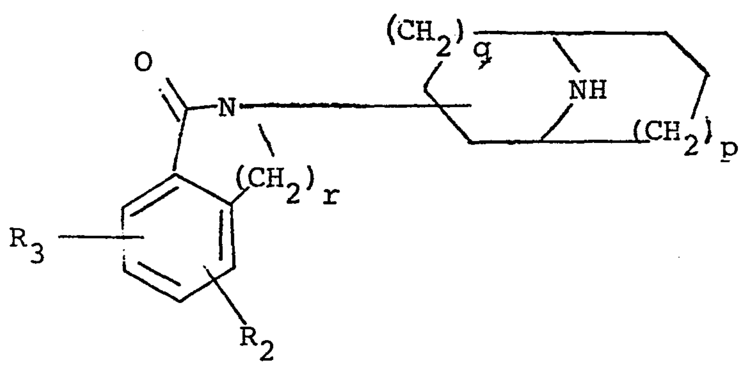

(XIV)

which may then be reacted with compound $Q_3R_4$ wherein $R_4$ is as defined in formula (I) and $Q_3$ is a group or atom readily displaced by a nucleophile, and optionally forming a pharmaceutically acceptable salt of the resulting compound of the formula (I).

Suitable values for $Q_3$ include Cl, Br, I, $OSO_2CH_3$ or $OSO_2C_6H_4pCH_3$.

Favoured values for $Q_3$ inclde Cl, Br and I.

Particularly suitably the compound $Q_3R_4$ is a benzyl halide such as benzyl bromide or benzyl chloride.

The reaction may be carried out under conventional alkylation conditions for example in an inert solvent such as dimethylformamide in the presence of an acid acceptor such as potassium carbonate. Generally the reaction is carried out at a non-extreme temperature such as at ambient or at a slightly elevated temperature.

It is preferred to interconvert $R_4$ in the compound of the formula (XII) before coupling with the compound of the formula (XI).

Conventional protection of the primary amine group is then preferred.

Substituents of $R_4$ may be interconverted, in particular such substituents as are representable by $L-R_{10}$ as defined hereinbefore whre $R_{10}$ may be interconverted.

Thus, when $R_{10}$ is carboxy or esterified carboxy it may be converted to esterified carboxy or carboxy respectively by conventional esterification or de-esterification reactions as appropriate.

$R_{10}$ hydroxy is derivable from $R_{10}$ $C_{1-4}$ alkoxy when $R_1$ is not $C_{1-4}$ alkoxy or from $R_{10}$ in-vivo hydrolysable acyloxy; and $L-R_{10}$ $C_{1-4}$ hydroxyalkyl may be prepared from $L-R_{10}$ carboxy or carboxy $C_{1-3}$ alkyl of the same number of carbon atoms, optionally esterified.

Conversion of $R_{10}C_{1-4}$ alkoxy to hydroxy is conveniently effected by conventional methods, such as by warming with aqueous hydrobromic acid. Alternatively this may be carried out by treatment with pyridine hydrochloride, boron tribormide or boron triiodide or iodotrimethylsilane.

When $R_{10}$ is in vivo hydrolysable acyloxy as defined conversion may be effected conventionally, for example by acidic or basic hydrolysis.

When $L-R_{10}$ is carboxy or carboxy $C_{1-3}$ alkyl as defined, optionally esterified, conversion to $L-R_{10}$ $C_{1-4}$ hydroxyalkyl may be effected by selective metal complex hydride reduction eg using lithium borohydride under conventional conditions.

Compounds of the formula (I) wherein $R_{10}$ is $C_{1-4}$ alkoxy or in vivo hydrolysable acyloxy are derivable by conventional O-alkylation or esterification reactions respectively from hydroxy $R_{10}$ compounds.

For alkoxy derivatives, the reaction may be carried out under conventional O-alkylation conditions, using a compound of the formula $R_{11}^{1}Q$ where $R^{1}_{11}$ is $C_{1-4}$ alkyl and Q is a group readily displaceable by a nucleophile. Suitable examples of Q include halide such as Cl, Br or I or labile acyloxy groups such as $OSO_2CH_3$ or $OSO_2.C_6H_4.p\ CH_3$.

The reaction is generally effected in an inert solvent, at a non-extreme temperature such as ambient or slightly elevated temperature, for example solvent reflux temperature.

For in-vivo hydrolysable esters, the reaction may be carried out under conventional esterification conditions using a compound of the formula $R^2_{11}W$ wherein $R^2_{11}$ is an acyl group capable of forming an in-vivo hydrolysable acyloxy group and W is a group which facilitates ester formation.

When W is halide the reaction is generally carried out in the presence of a base; when W is hydroxyl it is generally effected in the presence of a dehydrating agent such as dicyclohexylcarbodiimide, in an inert solvent, at a non-extreme temperature such as ambient or slightly elevated temperature, for example solvent reflux temperature.

It will be appreciated by the skilled man that O-alkylation or O-acylation may also lead to amine N-alkylation or N-acylation, unless any nitrogen atom(s) is/are protected, as necessary.

For example, this may be conveniently done by carrying out acylation in the presence of a strong acid such as trifluoroacetic acid, so that any amine function is protected by protonation. The acid may conveniently also act as the solvent.

It will of course be appreciated that all the foregoing conversions may also be effected on corresponding variables in those reaction products of the coupling of the compounds of formulae (XI) and (XII) which are not of formula (I).

The acid addition salts of compounds of the formula (I) may be prepared in entirely conventional manner by reacting a compound of the formula (I) in base form with the chosen acid.

The quaternary ammonium salts of the compounds of the formula (I) may be prepared in conventional manner for such salts, such as by reaction of the chosen compound of the formula (I) with a compound $R_8Y$ as defined. This reaction is suitably carried out in an appropriate solvent such as acetone, methanol, ethanol, dimethylformamide and the like, at ambient or raised temperature and pressure.

The nitrogen atom of the heterocycle side chain may also form an N-oxide to give an internal N-oxide salt of the compound of the formula (I). The N-oxides may be prepared in conventional manner such as by reaction of the chosen compound of the formula (I) with an organic per-acid, such as m-chloroperbenzoic acid. This reaction is suitably carried out at below-ambient in e.g. a chlorinated hydrocarbon.

From the foregoing it will be seen that the invention provides a number of other processes for the preparation of a compound of the formula (I) characterised by:

a) the reduction of a compound of the formula (XV):

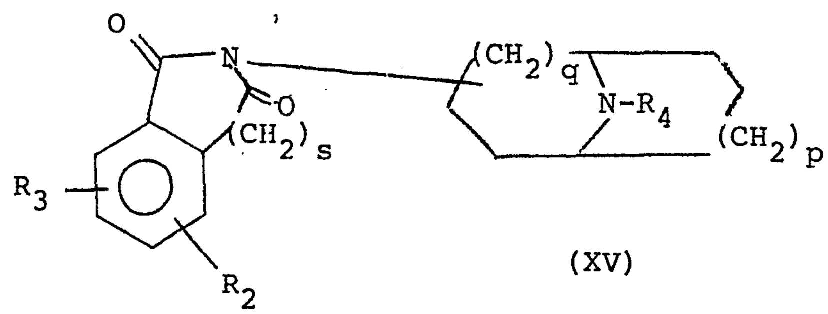

(XV)

b)or the reaction of a compound of the formula (XVI)

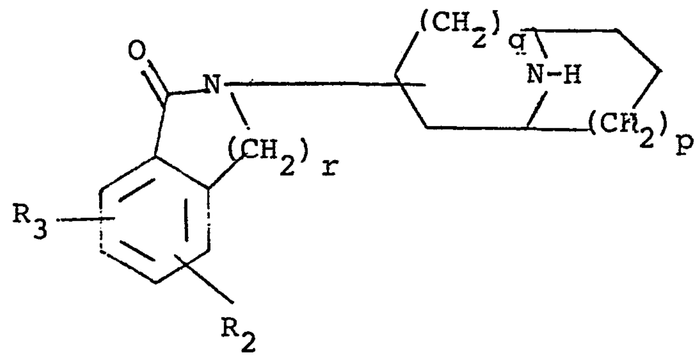

(XVI)

with a compound $Q_3R_4$.

Variables and reaction conditions are as hereinbefore defined and described.

As hereinbefore stated, the compounds of the formula (I) are dopamine antagonists.

The compounds of the present invention are dopamine antagonists and may generally be used in the treatment of emesis. Depending on their balance between peripheral and central action on the nervous system, they may also be used in the treatment of disorders relating to impaired gastro-intestinal motility, such as retarded gastric emptying, dyspepsia, flatulence, oesophagal reflux and peptic ulcer and/or in the treatment of disorders of the central nervous system, such as psychosis.

The invention therefore also provides a pharmaceutical composition comprising a compound of the formula (I), or a solvate or a pharmaceutically acceptable salt thereof, together with a pharmaceutically acceptable carrier. Such compositions may be adapted for oral or parenteral

administration, and as such may be in the form of tablets, capsules, oral liquid preparations, powders, granules, lozenges, reconstitutable powders, injectable and infusable solutions or suspensions; the compositions may also be in the form of suppositories. Normally, orally administrable compositions are preferred.

Tablets and capsules for oral administration may be in unit dose presentation form, and may contain conventional excipients such as binding agents, fillers, tabletting lubricants, disintegrants, and acceptable wetting agents. The tablets may be coated according to methods well known in normal pharmaceutical practice. Oral liquid preparations may be in the form of, for example, aqueous or oily suspensions, solutions, emulsions, syrups, or elixirs, or may be presented in a dry product for reconstitution with water or other suitable vehicle before use. Such liquid preparations may contain conventional additives such as suspending agents, emulsifying agents, non-aqueous vehicles (which may include edible oils), preservatives, and if desired conventional flavouring or colouring agents.

For parenteral administration, fluid unit dosage forms are prepared utilising the compound of the formula (I) and a sterile vehicle. The compound, depending on the vehicle and concentration used, can be either suspended or dissolved in the vehicle. In preparing solutions the compound can be dissolved for injection and filter sterilized before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilized by exposure to ethylene oxide before suspending in the sterile vehicle.

Advantageously, a surfactant or wetting agent is included in the composition to facilitate uniform distribution of the compound.

As is common practice, the compositions will usually be accompanied by written or printed directions for use in the medical treatment concerned.

The invention further provides a method of treatment of disorders in mammals including humans comprising the administration of an effective amount of a compound of the formula (I) or a solvate or pharmaceutically acceptable salt thereof to the sufferer. The "effective amount" will depend in the usual way on a number of factors such as the nature and severity of the malady to be treated, the weight of the sufferer, and the actual compound used.

It will of course be realised that the precise dosage used in the treatment of any of the hereinbefore described disorders will depend on the actual compound of the formula (I) used, and also on other factors such as the seriousness of the disorders.

However by way of illustration, unit doses will suitably contain 0.1 to 20 mgs of the compound of formula (I), for example 0.5 to 10 mgs.

Again by way of illustration, such unit doses will suitably be administered more than once a day, for example 2, 3, 4, 5 or 6 times a day, in such a way that the total daily dose is suitably in the range 0.01 to 10 mg/kg per day.

Compounds of the formula (I) have the ability to potentiate the effect of conventional analgesics in migraine treatment when administered concurrently with the analgesic.

Thus the invention provides a pharmaceutical composition comprising a compound of the formula (I) and an analgesic.

The compound of the formula (I) and the analgesic, such as aspirin or paracetamol, will be present in the composition in amounts generally similar to their usual effective dose.

The composition can be a combination product, for example a tablet or capsule containing both a compound of the formula (I) and an analgesic for oral administration, or a twin pack comprising the two active ingredients made up for separate administration.

The invention accordingly provides a method of treatment of migraine comprising the administration to the sufferer of a compound of the formula (I) and an analgesic.

The invention also provides a compound of formula (I) or a pharmaceutically acceptable salt thereof for use in treating CNS disorders or disorders related to impaired gastric motility in humans.

The following Examples illustrate the preparation of the compounds of formula (I) and the following Descriptions illustrate the preparation of intermediates thereto.

Description 1

[3β-N-(9-(4'-methylbenzyl)-9-azabicyclc [3.3.1] nonyl] phthalimide(D1)

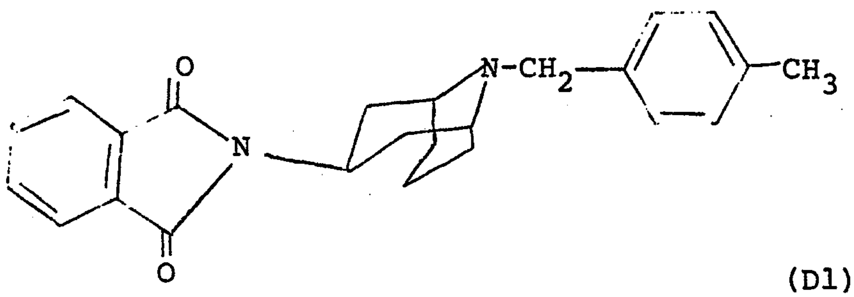

(D1)

Phthalic anhydride (0.74g) was dissolved in toluene (50 ml) and warmed to 50°C. 3β-amino-9-(4[1]-methylbenzyl) -9-azabicyclo (3.31) nonane (1.22g) was dissolved in toluene (20 ml) and added dropwise in 15 minutes. The reaction mixture was refluxed for 16 hours, the solvent evaporated and the residue heated to 200°C. After cooling, column chromatography on silica gel using ethyl acetate as eluant gave the desired product (1.0g, 53%).

Description 2

4-Chloro-o-toluic acid ethyl ester (D2)

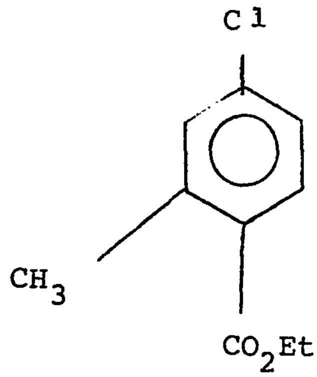

(D2)

To a refluxing mixture of m-chlorotoluene (38g) and aluminium chloride (45.9g) in carbon disulphide (300 ml) was added acetyl chloride (24.6 ml). The reaction mixture was refluxed for 1 hour, allowed to cool and poured into a mixture of ice (200 ml) and concentrated hydrochloric acid (200 ml). The solution was extracted with toluene and the organic extracts washed with water. The solvent was evaporated and the residue distilled to give a clear liquid (40 g, 79%), b.p. 84-86$^{o}$ (9 mm), consisting of a 1:1 mixture of 2-acetyl-5-chlorotoluene and 4-acetyl-5-chlorotoluene.

The mixture was added at room temperature to a solution of sodium hypobromite, prepared at 0$^{o}$C by dissolving sodium hydroxide (120 g) and bromine (38.2 ml) in water (515 ml). After stirring for 15 hours, sodium bisulfite (58.5 g) was added and the reaction mixture stirred for 30 minutes, washed with ether, acidified with concentrated hydrochloric acid and extracted with chloroform. Evaporation of the solvent gave a white solid (25 g, 61%), consisting of the two isomeric acids.

The mixture of acids was dissolved inethanol (250 ml), concentrated sulphuric acid (4 ml) added, and the solution refluxed for 48 hours. The solvent was evaporated, the residue dissolved in dichloromethane and washed with sodium bicarbonate solution and water. The solvent was evaporated to give the product, consisting of the two isomeric esters in a 1:1 mixture, as a pale yellow oil (25.6 g, 80%). Fractional distillation gave a mixture of the desired compound and 2-chloro-p-toluic acid ethyl ester in a ratio of 70:30. This mixture was used in the next stage without further purification.

Description 3

Ethyl 2-bromomethyl-4-chloro-benzoate (D3)

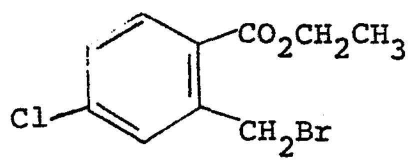

(D3)

The product (3 g) of the above reaction (D2) and N-bromosuccinimide (2.65 g) in carbon tetrachloride (50 ml) were refluxed for 3 hours. The solution was filtered and washed with dilute sodium hydroxide and water. The solvent was evaporated to give the product (3g, 72%) which was used in the next stage without further purification.

Description 4

2-Hydroxy-6-chloro-toluene (D4)

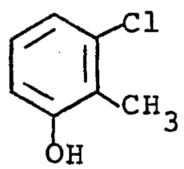

(D4)

A solution of 2-amino-6-chlorotoluene (14.15g) in 5.4% sulphuric acid (280ml) was cooled in ice and a solution of sodium nitrite (7.1g) in water (25ml) added slowly. After 15 minutes at room temperature, the reaction mixture was refluxed for 20 minutes. Subsequent steam distillation gave D4 (8.65g, 60%).

Description 5

2-Methoxy-6-chlorotoluene (D5)

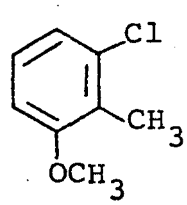

(D5)

A solution of 2-hydroxy-6-chlorotoluene (D4, 8.6g) in acetone (100ml) was stirred for 24 hours with dimethyl sulphate (7.6g) and potassium carbonate (10g). The solution was filtered and the solvent evaporated to give the crude product which was chromatographed on silica gel using ethyl acetate as eluant to give D5 (7g, 75%).

Description 6

3-Methoxy-2-methylbenzoic acid (D6)

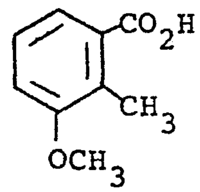

(D6)

A solution of 2-methoxy-6-chlorotoluene (D5, 7g) in dry tetrahydrofuran (30ml) was refluxed under nitrogen with magnesium turnings (1.26) and a crystal of iodine for 24 hours. The solution was allowed to cool and carbon dioxide bubbled through the solution for 2 hours. The solvent was evaporated and the residue dissolved in 10% sodium carbonate solution, acidified with dilute hydrochloric acid and extracted with ethyl acetate. Evaporation of the solvent gave D6 as a crystalline solid (5.6g, 76%) m.p. 145-7$^{o}$C.

Description 7

Methyl-3-methoxy-2-methylbenzoate (D7)

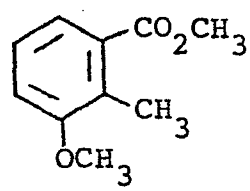

(D7)

A solution of 3-methoxy-2-methylbenzoic acid (D6, 5.7g) in dry methanol (200ml) with a few drops of concentrated sulphuric acid was refluxed for 24 hours. The solvent was evaporated and the residue dissolved in ethyl acetate, washed with 10% sodium carbonate, and the solvent evaporated to give D7 (5.4g, 89%).

Description 8

Methyl-2-bromomethyl-3-methoxybenzoate (D8)

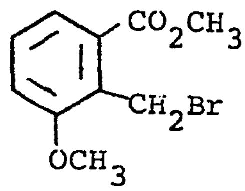

(D8)

The product (3g) of the above reaction (D7) and N-bromosuccinimide (2.9g) in carbon tetrachloride (50ml) were refluxed for 24 hours. The solution was filtered and washed with dilute sodium hydroxide and water.

The solvent was evaporated to give the product (3.2g, 74%) which was used in the next stage without further purification.

Desciption 9

3-Nitro-6-cyanotoluene (D9)

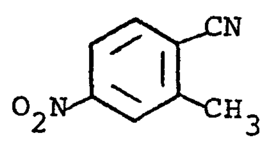

(D9)

3-Nitro-6-aminotoluene (10g) was dissolved, with warming, in a mixture of concentrated hydrochloric acid (17ml) and water (17ml). The mixture was cooled rapidly, ice (60g) was added, followed by a solution of sodium nitrite (5g) in water (20ml) in one portion. The solution was filtered and added dropwise to a solution of copper sulphate (24g) and potassium cyanide (26g) in water (150ml) at $70^{o}$. The reaction mixture was maintained at $60^{o}$ for 1 hour, cooled and filtered. The solid material was extracted with ethyl acetate and the solvent evaporated to give crude D9 (9.4g, 88%) which was converted to D10 without further purification.

Description 10

2-Methyl-4-nitrobenzoic acid (D10)

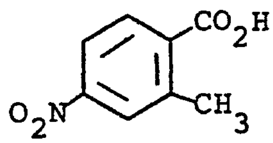

(D10)

A solution of 3-nitro-6-cyanotoluene (9.4g) in 65% sulphuric acid (100ml) was refluxed for 1 hour, allowed to cool and poured into ice. The aqueous solution was extracted with ethyl acetate and the solvent evaporated to give the crude product which was chromatographed on silica gel, using ethyl acetate as eluant, to give D10 (7g, 67%).

Description 11

Ethyl-2-methyl-4-nitrobenzoate (D11)

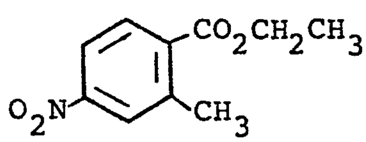

(D11)

A solution of 2-methyl-4-nitrobenzoic acid (7g) in ethanol (250ml) with a few drops of concentrated sulphuric acid was refluxed for 24 hours.

The solvent was evaporated and the residue dissolved in ethyl acetate, washed with 10% sodium carbonate, and the solvent evaporated to give D11 (6.8g, 85%).

Description 12

Ethyl-2-bromomethyl-4-nitrobenzoate (D12)

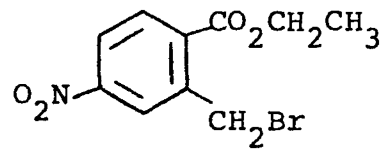

(D12)

The product (3.5g) of the above reaction (D11) and N-bromosuccinimide (2.9g) in carbon tetrachloride (50ml) were refluxed for 5 hours. The solution was filtered and washed with dilute sodium hydroxide and water. The solvent was evaporated to give D12 (4.5g, 92%) which was converted to D13 without further purification.

Description 13

5-Nitro-2-[3β-N-(9-(4[1]-fluorobenzyl)-9-azabicyclo[3.3.1.]nonyl)]phthalimidine (D13)

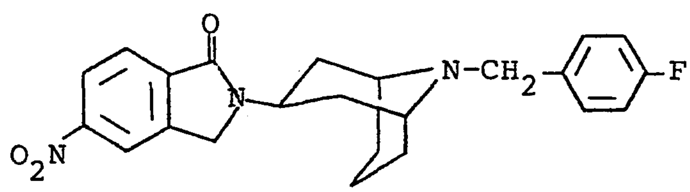

(D13)

3β-Amino-9-(4[1]-fluorobenzyl)-9-azabicyclo[3.3.1]nonane (2.1g) was refluxed in toluene (75ml) and the product (D12, 2.7g) from the above reaction in toluene (25ml) was added over 15 minutes. The reaction mixture was refluxed for 2 hours, cooled and washed with dilute sodium hydroxide and water. The solvent was evaporated and the crude product washed with ether to give D13 (3.1g, 84%) which was used in the next stage without further purification.

Description 14

5-Nitro-2-[3β-N-(9-(4[1]-methylbenzyl)-9-azabicyclo[3.3.1] nonyl)phthalimidine (D14)

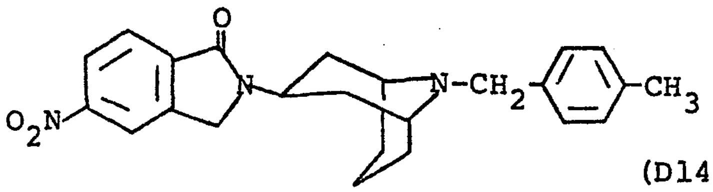

(D14)

By a procedure similar to that described in Description 13 but using 3β-amino-9-(4[1]-methylbenzyl)-9-azabicyclo [3.3.1]nonane, compound D14, (71%), was obtained.

Description 15

5-Chlorosulphonyl-2-methylbenzoic acid (D15)

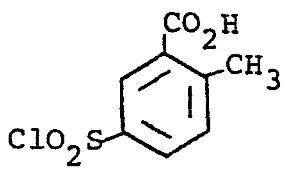

(D15)

2-Methylbenzoic acid (5g) was added portionwise to chlorosulphonic acid (13mls) while keeping the temperature of the mixture below 5$^{o}$C. The reaction mixture was then warmed to 55$^{o}$C and maintained at this temperature for 2 hours before cooling and pouring into ice-water. The precipitated product was filtered and dried in vacuo to give D15 (8g) which was converted to D16 immediately.

Description 16

2-Methyl-5-dimethylaminosulphonylbenzoic acid (D16)

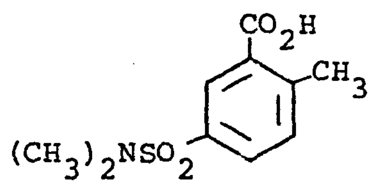

(D16)

5-Chlorosulphonyl-2-methylbenzoic acid (D15, 4.9g) was added in small portions to a cooled solution of 33% dimethylamine (20mls) and the resulting solution was allowed to stand overnight. Acidification with 5N hydrochloric acid gave a precipitate which was filtered, washed with water and dried over potassium hydroxide in vacuo to give D16 (3.75g, 73%) m.p. 180-182°C.

Description 17

Ethyl 2-methyl-5-dimethylaminosulphonylbenzoate (D17)

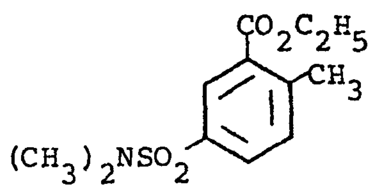

(D17)

A mixture of 2-methyl-5-dimethylaminosulphonylbenzoic acid (D16, 5g) and thionyl chloride (25mls) was heated under reflux for 1 hour before the removal of excess thionyl chloride in vacuo.

Ethanolic hydrogen chloride (30mls) was added to the residue and the reaction mixture stirred at room temperature overnight before being poured into water. The precipitate formed was filtered and dried in vacuo to give D17 (5g, 80%) m.p. 87-90°C.

Description 18

Ethyl 2-bromomethyl-5-dimethylaminosulphonylbenzoate (D18)

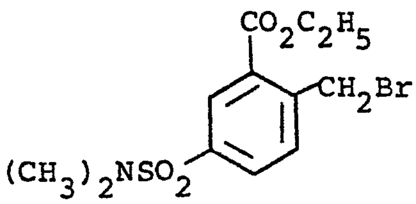

(D18)

N-bromosuccinimide (1.97g) and 2,2[1] bis azaisobutyronitrile (20mgs) were added to a solution of ethyl 2-methyl -5-dimethylaminosulphonyl benzoate (D17, 3g) in carbon tetrachloride (30mls) and the mixture was heated under reflux for 15 hours.

The reaction mixture was diluted with methylene chloride before being washed with water, and the organic layer was dried, filtered and evaporated in vacuo to give a 1:1 mixture (3.96g) of D18 and unreacted D17. This mixture was used without purification.

Example 1

[3β-N-(9-(4'-methylbenzyl)-9-azabicyclo(3.3.1)nonyl]phthalimidine (E1)

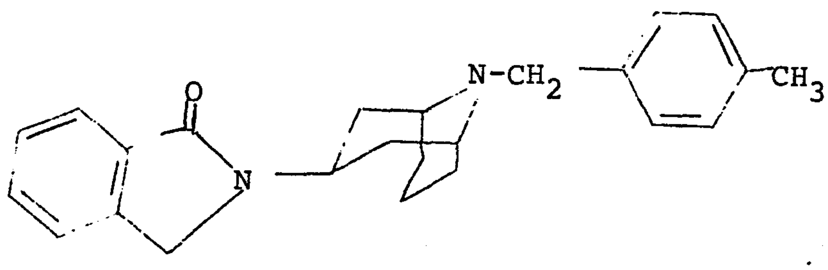

(E1)

3β-N-(9-(4$^1$-methylbenzyl)-9-azabicyclo (3.3.1)nonyl phthalimide (1.0g)was dissolved in acetic acid (10 ml). Tin (0.75g)was added and the mixture cooled in ice. Concentrated hydrochloric acid (1.5 ml) was added dropwise and the reaction mixture refluxed for 16 hours. The solution was evaporated to dryness and the residue dissolved in dilute hydrochloric acid, basified with potassium carbonate and extracted with dichloromethane. Evaporation of the solvent gave the crude product which was chromatographed on silica gel using ethyl acetate/petol as eluant to give the product (0.2g, 25% mp 89-91$^{O}$C.

Example 2

5-chloro-2-[3β-N-(9-(4'-methylbenzyl)-9-azabicyclo [3.3.1] nonyl] -phthalimidine (E2)

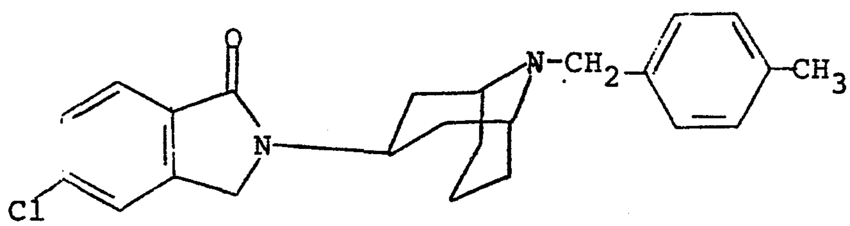

(E2)

3β-amino-9-(4'-methylbenzyl)-9-azabicyclo(3.3.1)nonane (2g) was refluxed in toluene (50 ml) and the product (D3) (3g) from the above reaction in toluene (15 ml) added over 15 minutes. The reaction mixture was refluxed for 1 hour, cooled and washed with dilute sodium hydroxide and water. The solvent was evaporated to give the crude product which was chromatographed on silica gel using ethyl acetate as eluant to give the desired product. Recrystallisation from ethyl acetate gave the pure product (0.6g, 20%) mp 176-8°C.

Example 3

5-chloro-2-[3β-N-(9-(4[1]-fluorobenzyl)-9-azabicyclo [3.3.1.] -nonyl]-phthalimidine (E3)

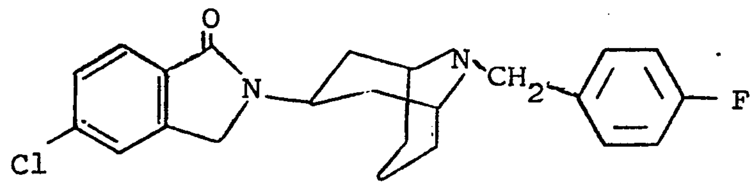

(E3)

By a procedure similar to that described in Example 2 but using 3β-amino-9-(4[1]flurobenzyl)-9-aza-bicyclo [3.3.1]-non ne, compound 3, (24%) m.p. 210-2$^{o}$ was obtained.

Example 4

4-Methoxy-2-[3β-N-(9-(4[1]-fluorobenzyl)-9-azabicyclo[3.3.1] nonyl)]-phthalimidine (E4)

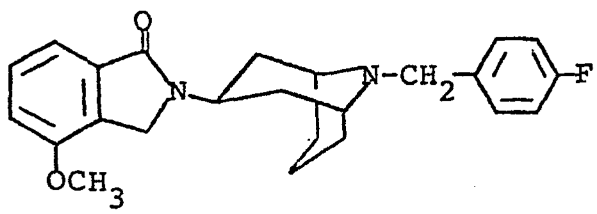

(E4)

3β-Amino-9-(4[1]-fluorobenzyl)-9-azabicyclo[3.3.1]nonane (1.4g) was refluxed in toluene (50ml) and the product D8 (1.5g) from the above reaction in toluene (15ml) added over 15 minutes. The reaction mixture was refluxed for 1 hour, cooled and washed with dilute sodium hydroxide and water. The solvent was evaporated to give the crude product which was chromatographed on silica gel using ethyl acetate as eluant to give the desired product. Recrystallisation from ethyl acetate gave E4 (0.7g, 31%) m.p. 186-8°C.

Example 5

5-Amino-2-[3β-N-(9-(4[1]-fluorobenzyl)-9-azabicyclo[3.3.1]nonyl)]phthalimidine (E5)

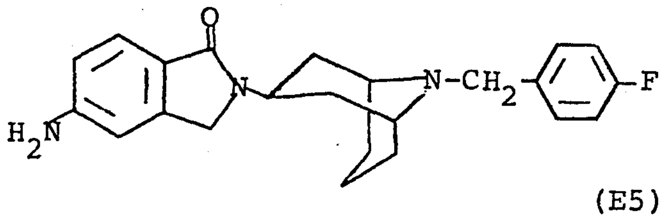

(E5)

The product (1.5g) from the above reaction (D13) in ethanol (100ml) was hydrogenated in the presence of Raney nickel catalyst. The solution was filtered and the solvent evaporated to give the crude product which was chromatographed on silica gel using ethyl acetate as eluant to give the desired product. Recrystallisation from ethyl acetate gave the pure product (0.4g, 29%) m.p. 231-3°.

Example 6

5-Amino-2-[3β-N-(9-(4[1]-methylbenzyl)-9-azabicyclo[3.3.1]nonyl)]phthalimidine (E6)

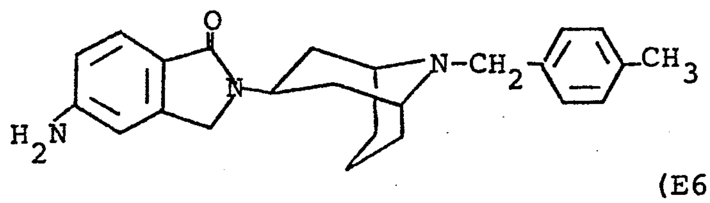

(E6)

The product (4g) from the above reaction (D14) in ethanol (250ml) was hydrogenated in the presence of Raney nickel catalyst. The solution was filtered and the solvent evaporated to give the crude product which was chromatographed on silica gel using ethyl acetate as eluant to give the desired product. Recrystallisation from ethyl acetate gave E6 (2.7g, 72%) m.p. 229-31$^{o}$.

Example 7

5-Acetylamino-2-[3β-N-(9-(4[1]-methylbenzyl)-9-azabicyclo[3.3.1]nonyl)]phthalimidine (E7)

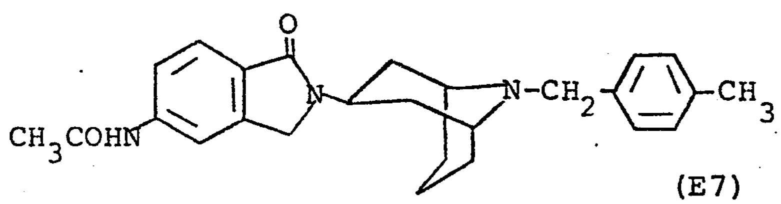

(E7)

The product (1g) from the above reaction (E6) in ethanol (50ml) was stirred at room temperature for 24 hours with excess acetic anhydride. The solvent was evaporated and the residue dissolved in ethyl acetate, washed with dilute sodium hydroxide solution, and the solvent evaporated to give E7 (0.7g, 65%).

Example 8

4-Chloro- and 6-Chloro-5-amino-2-[3β-N-(9-(4[1]-methylbenzyl)-9-azabicyclo[3.3.1]nonyl)]phthalimidine (E8)

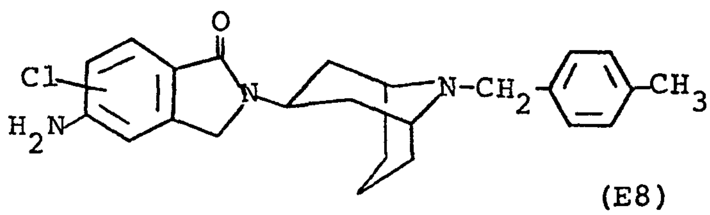

(E8)

The product (0.7g) from the above reaction (E7) was dissolved in glacial acetic acid (15ml) and 1 equivalent of chlorine in glacial acetic acid was added. The solution was stirred at room temperature for 4 hours, the solvent was evaporated and the residue was refluxed in ethanol (25ml) and concentrated hydrochloric acid (5ml) for 24 hours. The solvent was evaporated and the residue dissolved in ethyl acetate and washed with 10% potassium carbonate solution and water. The solvent was evaporated to give the crude product which was chromatographed on silica gel using ethyl acetate as eluant to give E8 (0.3g, 43%) as a 2:1 mixture of 4-chloro- and 6-chloro-5-amino-2-[3β-N-(9-(4[1]-methylbenzyl)-9-azabicyclo[3.3.1]nonyl)]phthalimidines.

Example 9

6-Dimethylaminosulphonyl-2-N-[3β(8-benzyl-8-azabicyclo [3.2.1]octyl)]phthalimidine (E9)

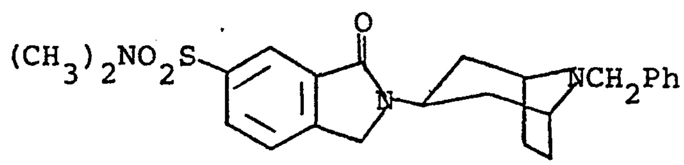

(E9)

3β-Amino-8-benzyl-8-azabicyclo[3.2.1]octane (0.6g) and crude D18 (1.0g) were dissolved in toluene and heated under reflux for 15 hours.

The mixture was then cooled, washed with sodium hydroxide solution, dried and evaporated to give an oil which was purified by column chromatography (silica, ethyl acetate) to give E9 (0.4g, 60%), m.p. 206-207°C.

PHARMACOLOGICAL DATA

The results in the following table are an illustration of the anti-psychotic activity of the present compounds as shown by Inhibition of Apormorphine Induced Climbing in the Mouse, a standard test.

Inhibition of apomorphine induced climbing in the mouse

The test is based on that described by Protais,P., Constantin, J.and Schwartz J.C. (1976), Psychopharmacology, 50, 1-6.

When mice are given a dose of 1 mg/kg apomorphine and then placed in an enclosed environment, such as an inverted wire cage, they are seen to climb around the walls. This behavioural phenomenon is though to be a consequence of the stimulation of post-synaptic Dopamine (D.A.) receptors in the nucleus accumbens. Inhibition of apomorphine induced climbing is therefore indicative of post-synaptic D.A. receptor blockade in accumbens.

Groups on 10 male CD1 mice, weighing 25-30 g were pretreated orally with either graded doses of the test compound or vehicle, at appropriate time intervals before the subcutaneous administration of a sub-maximal dose of apomorphine (1 mg/kg). Immediately after the apomorphine injection the mice were placed in wire 'climbing cages' and each animal was scored for climbing behaviour at 10 and 20 minutes post apomorphine as follows:-

Four paws on cage floor = 0
Fore paws on cage wall = 1
Four paws on cage wall = 2

The total score was calculated for each group of mice and expressed as a percentage inhibition of climbing.

$$\%\ \text{inhibition} = 100 - \frac{\text{Total score for test compound} \times 10}{\text{Total score for apomorphine control}}$$

ED50's and fiducial limits were calculated according to the method of Litchfield and Wilcoxon, the ED50 being the dose that produced a 50% inhibition of apomorphine-induced climbing.

The table shows the dose for 50% inhibition at ½ hour post dosing po.

| Compound No. | $ED_{50}$ mg/kg |
|---|---|
| E1 | 1.1 |
| E2 | 0.5 |
| E3 | 1.2 |
| E4 | 65% inhibition at 10mg/kg |
| E5 | 0.6 |
| E6 | 0.3 |
| E8 | 0.5 |
| E9 | 3.0 (subcutaneous dosing) |

<u>Toxicity</u>

No toxic effects were observed in the above tests.

Claims

1. A compound of the formula (I) or a pharmaceutically acceptable salt and/or solvate and/or N-oxide thereof:

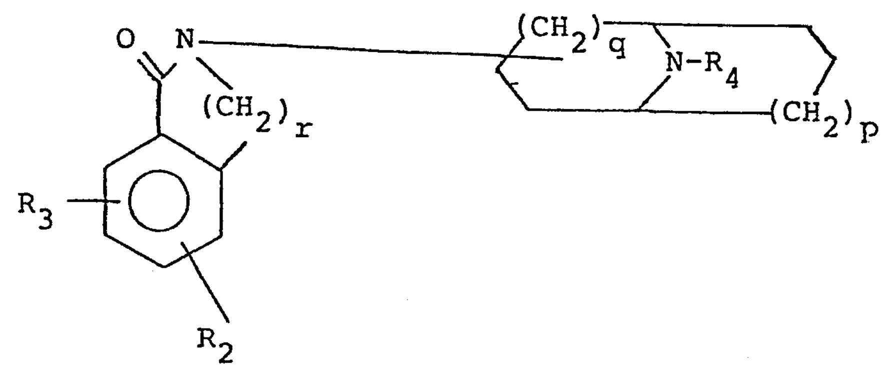

(I)

wherein:

$R_2$ and $R_3$ are the same or different and are hydrogen, halogen, $CF_3$, $C_{1-6}$ alkyl, $C_{1-7}$ acyl, $C_{1-7}$ acylamino or amino, aminocarbonyl or aminosulphonyl optionally substituted by one or two $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups either of which phenyl moieties may be substituted by one or more halogen, trifluoromethyl, $C_{1-6}$ alkoxy or nitro groups or N-substituted by $C_{4-5}$ polymethylene, $C_{1-6}$ alkylsulphonyl, $C_{1-6}$ alkylsulphinyl, $C_{1-6}$ alkoxy $C_{1-6}$ alkylthio; hydroxy or nitro; or $R_2$ and $R_3$ taken together are methylenedioxy or ethylenedioxy;

$R_4$ is $C_{1-7}$ alkyl or a group $-(CH_2)_sR_6$ where s is 0 to 2 and $R_6$ is a $C_{3-8}$ cycloalkyl group, or a group $-(CH_2)_tR_7$ where t is 1 or 2 and $R_7$ is a phenyl group optionally substituted by one or two substituents selected from $C_{1-4}$ alkoxy, trifluoromethyl and halogen, carboxy, esterified carboxy, or $C_{1-4}$ alkyl optionally substituted by hydroxy, $C_{1-4}$ alkoxy in vivo hydrolysable acyloxy, carboxy, or esterified carboxy; or a thienyl group;

p and q are independently 0 to 2; and

r is 1 or 2.

2. A compound according to claim 1 of formula (IV):

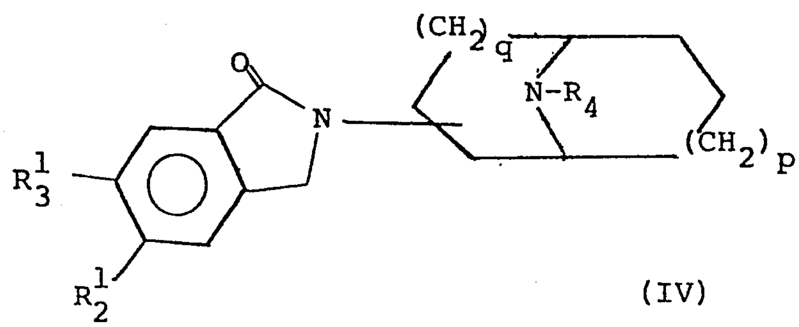

(IV)

wherein $R_2^1$ is hydrogen, halogen, or amino and $R_3^1$ is hydrogen or halogen.

3. A compound according to claim 2 of the formula (VIII):

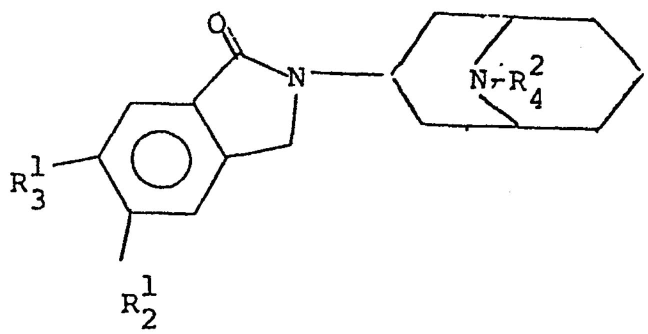

(VIII)

wherein $R_4^2$ is a group $-(CH_2)_tR_7^1$ wherein t is 1 or 2 and $R_7^1$ is optionally substituted phenyl as defined in claim 1; or 2-thienylmethyl; and $R_2^1$ and $R_3^1$ are as defined in claim 2.

4. A compound according to claim 2 or 3 wherein $R_2^1$ is hydrogen, bromo, chloro or amino and $R_3^1$ is hydrogen.

5. A compound according to claim 2 or 3 wherein $R_2^1$ is amino and $R_3^1$ is chloro.

6. A compound according to any one of claims 1 to 5 wherein $R_4$ or $R_4^2$ is benzyl, 4-methybenzyl, 4-fluorobenzyl or 4-chlorobenzyl.

7. [3β-N-(9-(4'-methylbenzyl)-9-azabicyclo(3.3.1) nonyl)]phthalimidine,

5-chloro-2-[3β-N-(9-(4'-methylbenzyl)-9-azabicyclo (3.3.1.) nonyl)]-phthalimidine,

5-chloro-2-[3β-N-(9-(4'-fluorobenzyl)-9-azabicyclo (3.3.1.) nonyl)]-phthalimidine,

5-amino-2-[3β-N-(9-(4'-fluorobenzyl)-9-azabicyclo (3.3.1.) nonyl)]phthalimidine,

5-amino-2-[3β-N-(9-(4'-methylbenzyl)-9-azabicyclo (3.3.1.) nonyl)]phthalimidine

or

6-chloro-5-amino-2-[3β-N-(9-(4'-methylbenzyl) -9-azabicyclo (3.3.1.) nonyl)]phthalimidine

8. A process for the preparation of a compound according to any one of the claims 1 to 7 characterised by reacting a compound of formula (XI):

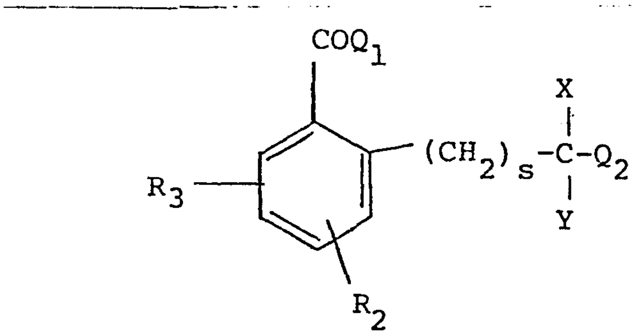

(XI)

wherein X is H and Y is H; or X and Y together form an oxo group; $Q_1$ and $Q_2$ are independently leaving groups; or together are O; s is O or 1; and the remaining variables are as in claim 1, with a compound of formula (XII);

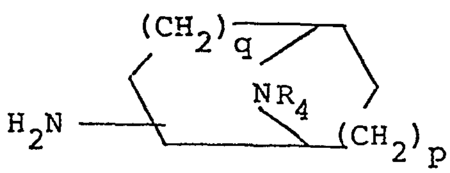

(XII)

wherein the variables are as defined in claim 1 and thereafter as necessary reducing X and Y when an oxo group to X and Y both hydrogen; and if desired or necessary converting a group $R_2$ or $R_3$ in the thus formed compound to another group $R_2$ or $R_3$ respectively; converting $R_4$ when hydrogen to another $R_4$; and optionally forming a pharmaceutically acceptable salt of the resultant compound of the formula (I).

9. A pharmaceutical composition comprising a compound according to any one of claims 1 to 7 or a pharmaceutically acceptable salt thereof, and a pharmaceutically acceptable carrier.

10. A compound according to any one of claims 1 to 7 or a pharmaceutically acceptable salt thereof for use in treating CNS disorders in humans.